# EUROPEAN PATENT APPLICATION

(11) **EP 4 825 321 A1**
(43) Date of publication of application: **21.10.2026**
(21) Application number: 25171253.5
(22) Date of filing: 17.04.2025
(51) Int. Cl.: A61B 6/02, A61B 6/46, A61B 6/00, H04N 13/366, H04N 13/383

(54) **MEDICAL IMAGING APPARATUS, COMPUTER-IMPLEMENTED METHOD, COMPUTER-READABLE STORAGE MEDIUM AND COMPUTER PROGRAM PRODUCT**

(71) Applicant: Siemens Healthineers AG, 91301 Forchheim (DE)
(72) Inventor: Pantano, Matteo, 81379 München (DE); Wang, Jian, 91301 Forchheim (DE)
(74) Representative: Siemens Healthineers Patent Attorneys

(57) **Abstract**

The present invention relates to a medical imaging apparatus for enhancing depth perception during medical image guidance, the medical imaging apparatus comprising: an autostereoscopic display, an image-acquisition module, a first registration module, an image-derivation module, a projection unit and a second registration module. In the present invention, it further relates to a method for enhancing depth perception during medical image guidance, a non-transitory computer-readable storage medium and a computer program product.

## Description

The present invention relates to a medical imaging apparatus for enhancing depth perception during medical image guidance. The present invention further relates to a computer-implemented method in particular employed in the medical imaging apparatus, a non-transitory computer-readable storage medium and a computer program product.

In endovascular interventions, digitally subtracted angiographs, DSA, images are commonly acquired and used to create a mask image as a roadmap for a medical device navigation. Those DSA images are usually live images. DSA is a fluoroscopy technique employed in interventional radiology to enhance the visualization of blood vessels within a bony structures or dense soft tissue. This process involves the use of a contrast medium, where a pre-contrast image or mask is subtracted from subsequent images after the contrast medium has been introduced into a region of interest. However, two-dimensional, 2D, roadmap images do not contain depth information, which may hide a variety of valuable diagnostic or therapeutic information during the medical device navigation. To enhance depth perception during the medical device navigation, three-dimensional, 3D, DSA images of the region of interest can be acquired and overlaid onto a live fluoroscopy image to create 3D roadmap. The 3D roadmap may give a better depth perception but obscures the live image content, which is critical for interventional guidance. To make the live image content more prominent, the transparency of the 3D overlay is often increased, although this reduces the depth perception it provides.

Therefore, there is an unmet need to provide an improved solution to enhance the depth perception during medical image guidance.

One of previous solutions to enhance the depth perception during the medical image guidance is to employ biplane systems to acquire 2D roadmap images from two different angulations simultaneously. Biplane roadmaps may restore the 3D geometrical information by combing two viewing angles. However, this doubles the radiation dose and requires the clinicians to mentally map the information from two separate images to reconstruct 3D information. Another approach to enhance the depth perception during the medical image guidance is to adapt a 3D visualization on 2D roadmap images while preserving the live image content. While blending 2D and 3D visualization, it is possible to integrate color coded hues or alike to map the depth information to the 2D view. However, this approach remains limitation of the depth perception due to the inherent nature of the 2D images.

DE 10 2012 200 536 A1 disclose a method for superimposed display of medical images, involves superimposing fluoroscopy image and reference image and visualizing contour of three-dimensional structures of object in reference image.

Against this background, the problem addressed by the present invention is that of providing a medical imaging apparatus for enhancing depth perception during medical image guidance.

This problem is solved according to the invention by a medical imaging apparatus having the features of claim 1 and/or a computer-implemented method for enhancing depth perception during medical image guidance having the features of claim 13 and/or by a non-transitory computer-readable storage medium having the features of claim 14 and/or a computer program product having the features of claim 15.

According to a first aspect, the present disclosure presents a medical imaging apparatus for enhancing depth perception during medical image guidance, the medical imaging apparatus comprising: an autostereoscopic display configured to display a stereoscopic medical image, wherein the stereoscopic medical image integrated by at least two autostereoscopic medical images; an image-acquisition module coupled to the autostereoscopic display and configured to capture and/or receive a 2D medical image of a region of interest under an acquisition angulation, and to capture and/or receive a 3D medical image of the region of interest, wherein the acquisition angulation of the 2D medical image is to be used as the first autostereoscopic orientation; a first registration module coupled to the image-acquisition module and configured to co-register the 2D medical image and the 3D medical image under the acquisition angulation to obtain a co-registration result; an image-derivation module coupled to the autostereoscopic display and the image-acquisition module and configured to compute the second autostereoscopic orientation according to a location and/or an orientation of eyes of an external interacted user relative to the autostereoscopic display and/or the first autostereoscopic orientation; a projection unit coupled to the first registration module and the image-derivation module and configured to compute a first volume projection of the 3D medical image under the acquisition angulation based on the co-registration result to obtain a first autostereoscopic image under the first autostereoscopic orientation, and configured to compute a second volume projection of the 3D medical image under the second autostereoscopic orientation; and a second registration module coupled to the projection unit and configured to co-register the 2D medical image and the 3D medical image under the second autostereoscopic orientation based on the second volume projection by performing a pixel shifting on the 2D medical image to obtain the second autostereoscopic image under the second autostereoscopic orientation.

According to a second aspect, the present disclosure presents a computer-implemented method for enhancing depth perception during medical image guidance, in particular employed in a medical imaging apparatus according to the first aspect, the method comprising: acquiring a 2D medical image and a 3D medical image of a region of interest under an acquisition angulation, wherein the acquisition angulation is configured to use as a first autostereoscopic orientation; co-registering the 2D medical image and the 3D medical image under the acquisition angulation to obtain a co-registration result; computing a second autostereoscopic orientation for an autostereoscopic display according to a location and/or an orientation of eyes of an external interacted user relative to the autostereoscopic display and/or the first autostereoscopic orientation; obtaining the first autostereoscopic image under the first autostereoscopic orientation by computing a first volume projection of the 3D medical image under the acquisition angulation based on the co-registration result; computing a second volume projection of the 3D medical image under the second autostereoscopic orientation; obtaining the second autostereoscopic image under the second autostereoscopic orientation by co-registering the 2D medical image and the 3D medical image under the second autostereoscopic orientation based on the second volume projection by performing a pixel shifting on the 2D medical image; and displaying a stereoscopic medical image integrated by a first autostereoscopic medical image under the first autostereoscopic orientation for the autostereoscopic display and a second autostereoscopic medical image under the second autostereoscopic orientation for the autostereoscopic display.

According to a third aspect, the present disclosure presents a non-transitory computer-readable storage medium storing instructions by a computer that the instructions are implemented in the medical imaging apparatus of the first aspect.

According to a fourth aspect, the present disclosure presents a computer program product including a computer program that is implemented in the medical imaging apparatus of the first aspect when program sections of the computer program are executed by a computer.

The present invention is based on the idea to provide a medical imaging apparatus for enhancing depth perception during medical image guidance. The medical imaging apparatus provides a 3D view of the roadmap content on one single monitor within one single image area.

The medical imaging apparatus comprises a 3D display and shows the 3D information on a 2D live image using the 3D display. The external interacted user does not need to switch between multiple image areas and mentally reconstruct the 3D geometry. The critical information from live 2D medical image may not hide due to the 3D display. The medical imaging apparatus provides an intuitive depth perception and a better visualization of the medical navigation.

The medical imaging apparatus comprises an autostereoscopic display, an image-acquisition module, a first registration module, an image-derivation module, a projection unit and a second registration module.

The autostereoscopic display is configured to display a stereoscopic medical image, which the stereoscopic medical image integrated by at least two autostereoscopic medical images.

The image-acquisition module is coupled to the autostereoscopic display. The image-acquisition module is configured to capture and/or receive a 2D medical image of a region of interest under an acquisition angulation. The image-acquisition module is configured to capture and/or receive a 3D medical image of a region of interest. A store unit may be coupled to the image-acquisition module. The image-acquisition module captures the images. The images captured by the image-acquisition module can be transferred to the store unit. The store unit receives the images and store the images. The store unit may be volatile or non-volatile. The store unit may also on a cloud memory. In addition, the store unit may be integrated with the image-acquisition module. Therefore, the image-acquisition module captures the images and both receives the images. The image-acquisition module may be further configured to receive the 2D and 3D medical image from an external source of data, for example, from a storage or memory of a local or a remote computer system. The image-acquisition module may be further configured to receive the 3D medical image from a computation result of the 3D medical image for the region of interest. In this invention, the acquisition angulation of the 2D medical image is to be used as a first autostereoscopic orientation.

The first registration module is coupled to the image-acquisition module and is configured to co-register the 2D medical image and the 3D medical image under the acquisition angulation to obtain a co-registration result. During the co-registration process, the depth of each 2D medical image pixel is matched to the depth map of the 3D medical image projected under the acquisition angulation.

The image-derivation module is coupled to the autostereoscopic display and the image-acquisition module. The image-derivation module is configured to compute the second autostereoscopic orientation according to a location and/or an orientation of eyes of an external interacted user relative to the autostereoscopic display and/or the first autostereoscopic orientation. The location and/or the orientation of eyes of an external interacted user relative to the autostereoscopic display may be obtained from an eye tracking system or other tracking systems mounted on or coupled to the autostereoscopic display. The second autostereoscopic orientation is different from the first autostereoscopic orientation.

The projection unit is coupled to the first registration module and the image-derivation module. The projection unit is configured to compute a first volume projection of the 3D medical image under the acquisition angulation based on the co-registration result to obtain a first autostereoscopic image under the first autostereoscopic orientation. Different volume rendering techniques can be applied to refine the correspondence between the 2D medical image and the 3D medical image, for example, depth map for depth recovery. The projection unit is further configured to compute a second volume projection of the 3D medical image under the second autostereoscopic orientation.

The second registration module is coupled to the projection unit. The second registration module is configured to co-register the 2D medical image and the 3D medical image under the second autostereoscopic orientation based on the second volume projection by performing a pixel shifting on the 2D medical image to obtain the second autostereoscopic image under the second autostereoscopic orientation. Different volume rendering techniques mentioned above can be applied here.

The first registration module and the second registration module may be from a same computation unit. The first registration module, the image-derivation module, the projection unit, and the second registration module may be from a same computation unit. The computation unit can comprise hardware elements and software elements, for example a microprocessor, a central processing unit, CPU, a graphical processing unit, GPU, a field programmable gate array, FPGA, or an application-specific integrated circuit, ASIC, or alike. The computation unit can be configured for multithreading, i.e., the computation unit can host different computation processes at the same time, executing the either in parallel or switching between active and passive computation processes.

A computer-implemented method for enhancing depth perception during medical image guidance, in particular employed in the proposed medical imaging apparatus is further provided. The method comprises acquiring a 2D medical image and a 3D medical image of a region of interest under an acquisition angulation, wherein the acquisition angulation is configured to use as a first autostereoscopic orientation; co-registering the 2D medical image and the 3D medical image under the acquisition angulation to obtain a co-registration result; computing a second autostereoscopic orientation for an autostereoscopic display according to a location and/or an orientation of eyes of an external interacted user relative to the autostereoscopic display and/or the first autostereoscopic orientation; obtaining the first autostereoscopic image under the first autostereoscopic orientation by computing a first volume projection of the 3D medical image under the acquisition angulation based on the co-registration result; computing a second volume projection of the 3D medical image under the second autostereoscopic orientation; obtaining the second autostereoscopic image under the second autostereoscopic orientation by co-registering the 2D medical image and the 3D medical image under the second autostereoscopic orientation based on the second volume projection by performing a pixel shifting on the 2D medical image; and displaying a stereoscopic medical image integrated by a first autostereoscopic medical image under the first autostereoscopic orientation for the autostereoscopic display and a second autostereoscopic medical image under the second autostereoscopic orientation for the autostereoscopic display.

A non-transitory computer-readable storage medium storing instructions by a computer that the instructions are implemented in the medical imaging apparatus and a computer program product including a computer program that is implemented in the medical imaging apparatus when program sections of the computer program are executed by a computer are also provided.

Advantageous configurations and developments emerge from the further dependent claims and from the description with reference to the figures of the drawings.

In a possible embodiment of the medical imaging apparatus, which the autostereoscopic display is further configured to display the stereoscopic medical image, which the stereoscopic medical image integrated by the first autostereoscopic medical image under the first autostereoscopic orientation for the autostereoscopic display and the second autostereoscopic medical image under the second autostereoscopic orientation for the autostereoscopic display. The autostereoscopic display relies on the fact that each human eye views the objects of the physical world from a slightly different angle. The brain may process these different perspectives to create the perception of depth. The autostereoscopic display employs this principle to create a 3D view for the human from 2D images without other supplementary instruments, such as glasses or virtual reality, VR, vision equipment or alike. The autostereoscopic display may be based on two-view systems, which provides a separate view for each eye using either parallax barriers or lenticular lenses in a thin layer on the front of the display.

In a possible embodiment of the medical imaging apparatus, which the image-acquisition module is configured such that the 2D medical image comprises a 2D roadmap image. The 2D medical image may be a 2D roadmap image used in endovascular interventions for the treatment of peripheral arterial and venous diseases and alike. To acquire a 2D roadmap image, the radiologist or the clinicians may need to acquire 2D digital subtraction angiography, DSA, showing the vessels of the patients, which is enhanced by injecting a radio-opaque contrast agent. The 2D DSA may be acquired by conventional 2D X-ray image in combination with contrast dye. For example, a regular 2D X-ray image may be taken at first. In the regular 2D X-ray image, bones and/or soft tissue or alike can be visualized on the 2D X-ray image. However, the vessels of the patients are not visible. For the further live catheter guidance or navigation, a contrast agent can be injected into the region of interest, and a second 2D X-ray image can be taken lively or static showing the contrast flow through the vessels, through which the vessels of the patients can be represented. The 2D roadmap or the 2D DSA can be obtained by the second 2D X-ray image, which is contrast-enhanced, subtracted from the regular 2D X-ray image that show bones and/or soft tissue or alike in the background. Those 2D roadmap images in endovascular interventions facilitates the development of minimally-invasive endovascular interventions.

In a possible embodiment of the medical imaging apparatus, which the image-acquisition module is configured such that the 3D medical image comprises a 3D DSA. The 3D medical image comprises the image information of the region of interest with regard to the length dimension, the width dimension and the depth dimension. The 2D roadmap does not provide the image information of the region of interest with regard to the depth dimension. The 3D DSA may be acquired following a similar procedure that used to acquire 2D DSA. The 3D DSA and the 2D DSA are recoding the same region of interest. The 3D DSA provides volumetric visualization of the region of interest, while the 2D DSA provides the visualization of the region of interest under an acquisition angulation.

In a possible embodiment of the medical imaging apparatus, which the first registration module is configured to co-register the 2D medical image and the 3D medical image under the acquisition angulation via a geometry calibration or an image registration. During the co-registration process, the depth of each 2D medical image pixel is matched to the depth map of the 3D medical image projected under the acquisition angulation. The 2D medical image may be a lively medical image. The geometry calibration may take the location of X-ray source, detector, and the patient and alike into account, which provides an accurate 3D reconstruction from the 2D medical image. The image registration may match 2D medical image and 3D medical image projected under the acquisition angulation according to their spatial correspondence. The co-registration process provides an initial alignment of depth between 2D medical image and 3D medical image projected under the acquisition angulation, which may enable later potential fine-tuning steps faster and more accurate.

In a possible embodiment of the medical imaging apparatus, which the first registration module and the projection unit are configured such that the first volume projection of the 3D medical image under the acquisition angulation is represented by a correspondence between the depth of each 2D medical image pixel and depth map of the 3D medical image projected under the acquisition angulation. Such correspondence is refined by applying at least one of the volume rendering techniques, VRT, as follow: feature-based VRT; intensity-based VRT; gradient-based VRT; and/or combinations thereof. The VRT may include feature-based, intensity-based or gradient-based methods or the combinations thereof. While feature-based registration relies on extracted features, intensity-based and gradient-based registration is performed by defining and optimizing a similarity measure based on the intensities or gradients of the images. The other volume rendering techniques may be also employed. This enables a fine adjustment to refine the alignment, positioning, or other parameters between the 2D medical image and the 3D medical image under the acquisition angulation.

In a possible embodiment of the medical imaging apparatus, which the second registration module is further configured to co-register the 2D medical image and the 3D medical image under the second autostereoscopic orientation based on the second volume projection by performing a depth-aware pixel shifting on the 2D medical image. The acquisition angulation of the 2D medical image for the region of interest can be used as the first autostereoscopic orientation. For the autostereoscopic display based on the two-view system, at least two views should be presented for the viewer's left and right eyes to see separately. After the co-register process and the volume projection process, the processed 2D medical image can be served as the first autostereoscopic image under the first autostereoscopic orientation, which offers the first view of the autostereoscopic display. In this embodiment, the 2D medical image for the region of interest captured and/or received can be served as a base line to derive the second autostereoscopic image. The second autostereoscopic orientation can be derived by the image-derivation module according to a location and/or an orientation of eyes of an external interacted user relative to the autostereoscopic display and/or the first autostereoscopic orientation. The projection unit computes the second volume projection of the 3D medical image under the second autostereoscopic orientation. The second registration module co-registers the 2D medical image and the 3D medical image under the second autostereoscopic orientation based on the second volume projection. In the process of obtaining the second autostereoscopic image, the depth information of the 2D medical image is converted or shifted based on the derived second autostereoscopic orientation. Therefore, the second autostereoscopic image can be obtained without the need to capture and/or receive a 2D medical image under the second view.

In a possible embodiment of the medical imaging apparatus, which the medical imaging apparatus comprises further an eye tracking system mounted on the autostereoscopic display, which the eye tracking system is configured to obtain the location and the orientation of the eyes of the interacted user relative to the autostereoscopic display. The eye tracking system comprises hardware and/or software components. The eye tracking system may comprise camera or optical sensor to tracks the pupil's position and the movement of the eye. The eye tracking system may use infrared light to tracks the pupil's position and the movement of the eye, which the infrared light reflects off the cornea and the retina, creating reflections that are tracked by the system. The software component of the eye tracking system may process the signals to calculate the location and the orientation of the eyes of the interacted user relative to the autostereoscopic display. The eye tracking system can measure the rotation of the eyes.

In a possible embodiment of the medical imaging apparatus, which the eye tracking system is further configured such that, when the location and/or the orientation of the eyes of the interacted user relative to the autostereoscopic display is updated, an updated first autostereoscopic orientation is computed based on a shift between a former location and/or the orientation of the eyes of the interacted user and an updated location and/or the orientation of the eyes of the interacted user. When the location and/or the orientation of the eyes of the interacted user relative to the autostereoscopic display is updated, the views of the autostereoscopic display may also be updated. The eye tracking system may be coupled to the image-acquisition module. In this case, the image-acquisition module is further configured to simulate an updated 2D medical image and an updated 3D image under the updated first autostereoscopic orientation. The image-derivation module may also derive an updated 2D medical image and an updated 3D image under an updated second autostereoscopic orientation based on the updated first autostereoscopic orientation. This can prevent the pseudo-visualization effects and the reversal of depth perception.

In a possible embodiment of the medical imaging apparatus, which the autostereoscopic display is further configured to display a stereoscopic medical image integrated by multiple autostereoscopic medical images under corresponding autostereoscopic orientations. The autostereoscopic display may be based on multi-view systems. In multi-view autostereoscopic displays, multiple adjacent views of the same 3D scene are provided in the viewing zone. For these displays, as the viewer's eyes move to the left and right, the view changes to provide a different perspective of the same scene. In this case, the image-derivation module may be further configured to compute the multiple autostereoscopic orientations based on the location and/or the orientation of eyes of an external interacted user relative to the autostereoscopic display and/or the first autostereoscopic orientation. In this case, the first autostereoscopic orientation can be referred to the base autostereoscopic orientation for the image-derivation module to calculate the multiple autostereoscopic orientations. This can enlarge the field of view for the observers.

In a possible embodiment of the medical imaging apparatus, which the autostereoscopic display is further configured to enable multi-observers to observe the region of interest displayed with the stereoscopic medical image. In this case, the eye tracking system or other tracking systems can not only track the location and/or the orientation of the eyes of an interacted user relative to the autostereoscopic display, but also track the location and/or the orientation of the eyes of other interacted users relative to the autostereoscopic display. The same computation unit that comprises the first registration module, the image-derivation module, the projection unit, and the second registration module may be a multi-tasking computation unit, which enables the computation of different views of the autostereoscopic display for different observers based on the captured and/or received 2D medical image.

In a possible embodiment of the medical imaging apparatus, which the apparatus comprises as least one of the following apparatuses: X-ray machine; mobile C-arms; and/or computed tomography angiography, CTA, machine. The X-ray machine emits X-ray beam to penetrate the regions of interest to obtain the projection data used for reconstructing a medical images. Mobile C-arms comprise also X-Ray generator. The C-shaped arm connects the generator and detector, allows movement horizontally, vertically and around the swivel axes, so that X-ray images of the patient are produced from many angles. The CTA machine uses a CT scanner to produce images. It may also include other machines capable of obtaining 2D and 3D medical images. The imaging apparatus proposed here enables cross-platform applications and improves the accessibility and flexibility of applications.

Where appropriate, the above-mentioned configurations and developments can be combined implementations can be combined with each other as desired, as far as this is reasonable. Further possible configurations, developments and implementations of the invention also include combinations, which are not explicitly mentioned, of features of the invention which have been described previously or are described in the following with reference to the embodiments. In particular, in this case, a person skilled in the art will also add individual aspects as improvements or supplements to the basic form of the present invention.

The present invention is described in greater detail in the following on the basis of the embodiments shown in the schematic figures of the drawings, in which:
- FIG. 1: illustrates a block diagram of an example of the medical image apparatus;
- FIG. 2: illustrates a block diagram of another example of the medical image apparatus;
- FIG. 3: illustrates a schematic diagram of an example of generating the stereoscopic medical image; and
- FIG. 4: illustrates a chart of an example of a method for enhancing depth perception.

The appended drawings are intended to provide further understanding of the embodiments of the invention. They illustrate embodiments and, in conjunction with the description, help to explain principles and concepts of the invention. Other embodiments and many of the advantages mentioned become apparent in view of the drawings. The elements in the drawings are not necessarily shown to scale.

In the drawings, like, functionally equivalent and identically operating elements, features and components are provided with like reference signs in each case, unless stated otherwise.

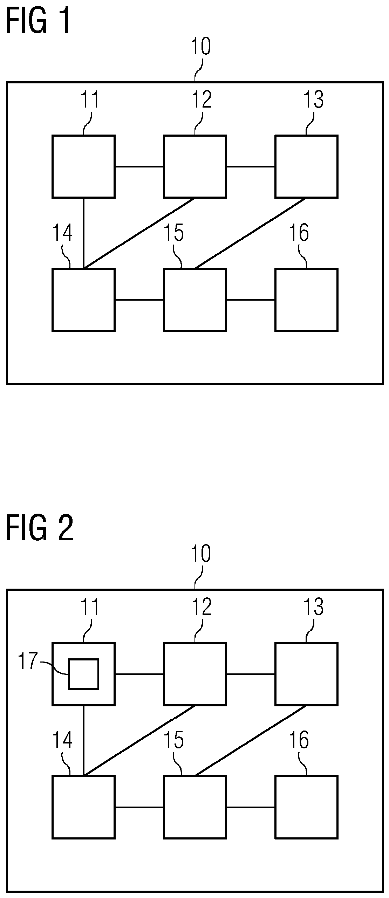

FIG. 1 illustrates a block diagram of an example of the medical image apparatus.

As shown in FIG. 1, the medical imaging apparatus 10 comprises an autostereoscopic display 11, an image-acquisition module 12, a first registration module 13, an image-derivation module 14, a projection unit 15 and a second registration module 16.

The autostereoscopic display 11 is configured to display a stereoscopic medical image, which the stereoscopic medical image integrated by at least two autostereoscopic medical images.

The image-acquisition module 12 is coupled to the autostereoscopic display 11. The image-acquisition module 12 is configured to capture and/or receive a 2D medical image 21 of a region of interest under an acquisition angulation 24. The image-acquisition module 12 is configured to capture and/or receive a 3D medical image 22 of a region of interest. A store unit may be coupled to the image-acquisition module 12. The image-acquisition module 12 captures the images. The images captured by the image-acquisition module 12 can be transferred to the store unit. The store unit receives the images and store the images. The store unit may be volatile or non-volatile. The store unit may also on a cloud memory. In addition, the store unit may be integrated with the image-acquisition module 12. Therefore, the image-acquisition module 12 captures the images and both receives the images. The image-acquisition module 12 may be further configured to receive the 2D and 3D medical image 21/22 from an external source of data, for example, from a storage or memory of a local or a remote computer system. The image-acquisition module 12 may be further configured to receive the 3D medical image 22 from a computation result of the 3D medical image 22 for the region of interest. In this invention, the acquisition angulation 24 of the 2D medical image 21 is to be used as a first autostereoscopic orientation 23.

The first registration module 13 is coupled to the image-acquisition module 12 and is configured to co-register the 2D medical image 21 and the 3D medical image 22 under the acquisition angulation 24 to obtain a co-registration result. During the co-registration process, the depth of each 2D medical image 21 pixel is matched to the depth map of the 3D medical image 22 projected under the acquisition angulation 24.

The image-derivation module 14 is coupled to the autostereoscopic display 11 and the image-acquisition module 12. The image-derivation module 14 is configured to compute the second autostereoscopic orientation 29 according to a location and/or an orientation of eyes of an external interacted user relative to the autostereoscopic display 11 and/or the first autostereoscopic orientation 23. The location and/or the orientation of eyes of an external interacted user relative to the autostereoscopic display 11 may be obtained from an eye tracking system (not shown in FIG. 1) or other tracking systems mounted on or coupled to the autostereoscopic display 11. The second autostereoscopic orientation 29 is different from the first autostereoscopic orientation 23.

The projection unit 15 is coupled to the first registration module 13 and the image-derivation module 14. The projection unit 15 is configured to compute a first volume projection 25 of the 3D medical image 22 under the acquisition angulation 24 based on the co-registration result to obtain a first autostereoscopic image 27 under the first autostereoscopic orientation 23. Different volume rendering techniques can be applied to refine the correspondence between the 2D medical image 21 and the 3D medical image 22, for example, depth map for depth recovery. The projection unit 15 is further configured to compute a second volume projection 26 of the 3D medical image 22 under the second autostereoscopic orientation 29.

The second registration module 16 is coupled to the projection unit 15. The second registration module 16 is configured to co-register the 2D medical image 21 and the 3D medical image 22 under the second autostereoscopic orientation 29 based on the second volume projection 26 by performing a pixel shifting on the 2D medical image 21 to obtain the second autostereoscopic image 28 under the second autostereoscopic orientation 29. Different volume rendering techniques mentioned above can be applied here.

The first registration module 13 and the second registration module 16 may be from a same computation unit. The first registration module 13, the image-derivation module 14, the projection unit 15, and the second registration module 16 may be from a same computation unit. The computation unit can comprise hardware elements and software elements, for example a microprocessor, a central processing unit, CPU, a graphical processing unit, GPU, a field programmable gate array, FPGA, or an application-specific integrated circuit, ASIC, or alike. The computation unit can be configured for multithreading, i.e., the computation unit can host different computation processes at the same time, executing the either in parallel or switching between active and passive computation processes.

The autostereoscopic display 11 is further configured to display the stereoscopic medical image, which the stereoscopic medical image integrated by the first autostereoscopic medical image 27 under the first autostereoscopic orientation 23 for the autostereoscopic display 11 and the second autostereoscopic medical image 28 under the second autostereoscopic orientation 29 for the autostereoscopic display 11. The autostereoscopic display 11 relies on the fact that each human eye views the objects of the physical world from a slightly different angle. The brain may process these different perspectives to create the perception of depth. The autostereoscopic display 11 employs this principle to create a 3D view for the human from 2D images without other supplementary instruments, such as glasses or virtual reality, VR, vision equipment or alike. The autostereoscopic display 11 may be based on two-view systems, which provides a separate view for each eye using either parallax barriers or lenticular lenses in a thin layer on the front of the display.

The image-acquisition module 12 is configured such that the 2D medical image 21 comprises a 2D roadmap image. The 2D medical image 21 may be a 2D roadmap image used in endovascular interventions for the treatment of peripheral arterial and venous diseases and alike. To acquire a 2D roadmap image, the radiologist or the clinicians may need to acquire 2D DSA showing the vessels of the patients, which is enhanced by injecting a radio-opaque contrast agent. The 2D DSA may be acquired by conventional 2D X-ray image in combination with contrast dye. For example, a regular 2D X-ray image may be taken at first. In the regular 2D X-ray image, bones and/or soft tissue or alike can be visualized on the 2D X-ray image. However, the vessels of the patients are not visible. For the further live catheter guidance or navigation, a contrast agent can be injected into the region of interest, and a second 2D X-ray image can be taken lively or static showing the contrast flow through the vessels, through which the vessels of the patients can be represented. The 2D roadmap or the 2D DSA can be obtained by the second 2D X-ray image, which is contrast-enhanced, subtracted from the regular 2D X-ray image that show bones and/or soft tissue or alike in the background. Those 2D roadmap images in endovascular interventions facilitates the development of minimally-invasive endovascular interventions.

The image-acquisition module 12 is configured such that the 3D medical image 22 comprises a 3D DSA. The 3D medical image 22 comprises the image information of the region of interest with regard to the length dimension, the width dimension and the depth dimension. The 2D roadmap does not provide the image information of the region of interest with regard to the depth dimension. The 3D DSA may be acquired following a similar procedure that used to acquire 2D DSA. The 3D DSA and the 2D DSA are recoding the same region of interest. The 3D DSA provides volumetric visualization of the region of interest, while the 2D DSA provides the visualization of the region of interest under an acquisition angulation 24.

The first registration module 13 is configured such to co-register the 2D medical image 21 and the 3D medical image 22 under the acquisition angulation 24 via a geometry calibration or an image registration. During the co-registration process, the depth of each 2D medical image 21 pixel is matched to the depth map of the 3D medical image 22 projected under the acquisition angulation 24. The 2D medical image 21 may be a lively medical image. The geometry calibration may take the location of X-ray source, detector, and the patient and alike into account, which provide an accurate 3D reconstruction from the 2D medical image 21. The image registration may match 2D medical image 21 and 3D medical image 22 projected under the acquisition angulation 24 according to their spatial correspondence. The co-registration process provides an initial alignment of depth between 2D medical image 21 and 3D medical image 22 projected under the acquisition angulation 24, which may enable later potential fine-tuning steps faster and more accurate.

The first registration module 13 and the projection unit 15 are configured such that the first volume projection 25 of the 3D medical image 22 under the acquisition angulation 24 is represented by a correspondence between the depth of each 2D medical image 21 pixel and depth map of the 3D medical image 22 projected under the acquisition angulation 24. Such correspondence is refined by applying at least one of the volume rendering techniques, VRT, as follow: feature-based VRT; intensity-based VRT; gradient-based VRT; and/or a combinations thereof. The VRT may include feature-based, intensity-based or gradient-based methods or the combinations thereof. While feature-based registration relies on extracted features, intensity-based and gradient-based registration is performed by defining and optimizing a similarity measure based on the intensities or gradients of the images. The other volume rendering techniques may be also employed. This enables a fine adjustment to refine the alignment, positioning, or other parameters between the 2D medical image 21 and the 3D medical image 22 under the acquisition angulation 24.

The second registration module 16 is further configured to co-register the 2D medical image 21 and the 3D medical image 22 under the second autostereoscopic orientation 29 based on the second volume projection 26 by performing a depth-aware pixel shifting on the 2D medical image 21. The acquisition angulation 24 of the 2D medical image 21 for the region of interest can be used as the first autostereoscopic orientation 23. For the autostereoscopic display 11 based on the two-view system, at least two views should be presented for the viewer's left and right eyes to see separately. After the co-register process and the volume projection process, the processed 2D medical image 21 can be served as the first autostereoscopic image under the first autostereoscopic orientation 23, which offers the first view of the autostereoscopic display 11. In this embodiment, the 2D medical image 21 for the region of interest captured or received or captured and received can be served as a base line to derive the second autostereoscopic image. The second autostereoscopic orientation 29 can be derived by the image-derivation module 14 according to a location and/or an orientation of eyes of an external interacted user relative to the autostereoscopic display 11 and/or the first autostereoscopic orientation 23. The projection unit 15 computes the second volume projection 26 of the 3D medical image 22 under the second autostereoscopic orientation 29. The second registration module 16 co-registers the 2D medical image 21 and the 3D medical image 22 under the second autostereoscopic orientation 29 based on the second volume projection 26. In the process of obtaining the second autostereoscopic image, the depth information of the 2D medical image 21 is converted or shifted based on the derived second autostereoscopic orientation 29. Therefore, the second autostereoscopic image can be obtained without the need to capture and/or receive a 2D medical image 21 under the second view.

The autostereoscopic display 11 is further configured to display a stereoscopic medical image integrated by multiple autostereoscopic medical images under corresponding autostereoscopic orientations. The autostereoscopic display 11 may be based on multi-view systems. In multi-view autostereoscopic displays 11, multiple adjacent views of the same 3D scene are provided in the viewing zone. For these displays, as the viewer's eyes move to the left and right, the view changes to provide a different perspective of the same scene. In this case, the image-derivation module 14 may be further configured to compute the multiple autostereoscopic orientations based on the location and/or the orientation of eyes of an external interacted user relative to the autostereoscopic display 11 and/or the first autostereoscopic orientation 23. In this case, the first autostereoscopic orientation 23 can be referred to the base autostereoscopic orientation for the image-derivation module 14 to calculate the multiple autostereoscopic orientations. This can enlarge the field of view for the observers.

The autostereoscopic display 11 is further configured to enable multi-observers to observe the region of interest displayed with the stereoscopic medical image. In this case, the eye tracking system (not shown in FIG. 1) other tracking systems can not only track the location and/or the orientation of the eyes of an interacted user relative to the autostereoscopic display 11, but also track the location and/or the orientation of the eyes of other interacted user relative to the autostereoscopic display 11. The same computation unit that comprises the first registration module 13, the image-derivation module 14, the projection unit 15, and the second registration module 16 may be a multi-tasking computation unit, which enables the computation of different views of the autostereoscopic display 11 for different observers based on the captured or received or captured and received 2D medical image 21.

The apparatus comprises as least one of the following apparatuses: X-ray machine; mobile C-arms; and/or computed tomography angiography, CTA, machine. The X-ray machine emits X-ray beam to penetrate the regions of interest to obtain the projection data used for reconstructing a medical image. Mobile C-arms comprise also X-Ray generator. The C-shaped arm connects the generator and detector, allows movement horizontally, vertically and around the swivel axes, so that X-ray images of the patient are produced from many angles. The CTA machine uses a CT scanner to produce images. It may also include other machines capable of obtaining 2D and 3D medical images 21/22. The imaging apparatus proposed here enables cross-platform applications and improves the accessibility and flexibility of applications.

The present invention is based on the idea to provide a medical imaging apparatus 10 for enhancing depth perception during medical image guidance. The medical imaging apparatus 10 provides a 3D view of the roadmap content on one single monitor within one single image area. The medical imaging apparatus 10 comprises a 3D display and shows the 3D information on a 2D live image using the 3D display. The external interacted user does not need to switch between multiple image areas and mentally reconstruct the 3D geometry. The critical information from live 2D medical image 21 may not hide due to the 3D display. The medical imaging apparatus 10 provides an intuitive depth perception and a better visualization of the medical navigation.

FIG. 2 illustrates a block diagram of another example of the medical image apparatus.

As shown in FIG. 2, the medical imaging apparatus 10 comprises an autostereoscopic display 11 11, an image-acquisition module 12, a first registration module 13, an image-derivation module 14, a projection unit 15 and a second registration module 16. In this example, the medical imaging apparatus 10 comprises further an eye tracking system 17 mounted on the autostereoscopic display 11.

The eye tracking system 17 is configured to obtain the location and the orientation of the eyes of the interacted user relative to the autostereoscopic display 11. The eye tracking system 17 comprises hardware and/or software components. The eye tracking system 17 may comprise camera or optical sensor to tracks the pupil's position and the movement of the eye. The eye tracking system 17 may use infrared light to tracks the pupil's position and the movement of the eye, which the infrared light reflects off the cornea and the retina, creating reflections that are tracked by the system. The software component of the eye tracking system 17 may process the signals to calculate the location and the orientation of the eyes of the interacted user relative to the autostereoscopic display 11. The eye tracking system 17 can measure the rotation of the eyes.

The eye tracking system 17 is further configured such, when the location and/or the orientation of the eyes of the interacted user relative to the autostereoscopic display 11 is updated, an updated first autostereoscopic orientation 23 is computed based on a shift between a former location and/or the orientation of the eyes of the interacted user and an updated location and/or the orientation of the eyes of the interacted user. When the location and/or the orientation of the eyes of the interacted user relative to the autostereoscopic display 11 is updated, the views of the autostereoscopic display 11 may also be updated. The eye tracking system 17 may be coupled to the image-acquisition module 12. In this case, the image-acquisition module 12 is further configured to simulate an updated 2D medical image 21 and an updated 3D image under the updated first autostereoscopic orientation 23. The image-derivation module 14 may also derive an updated 2D medical image 21 and an updated 3D image under an updated second autostereoscopic orientation 29 based on the updated first autostereoscopic orientation 23. This can prevent the pseudo-visualization effects and the reversal of depth perception.

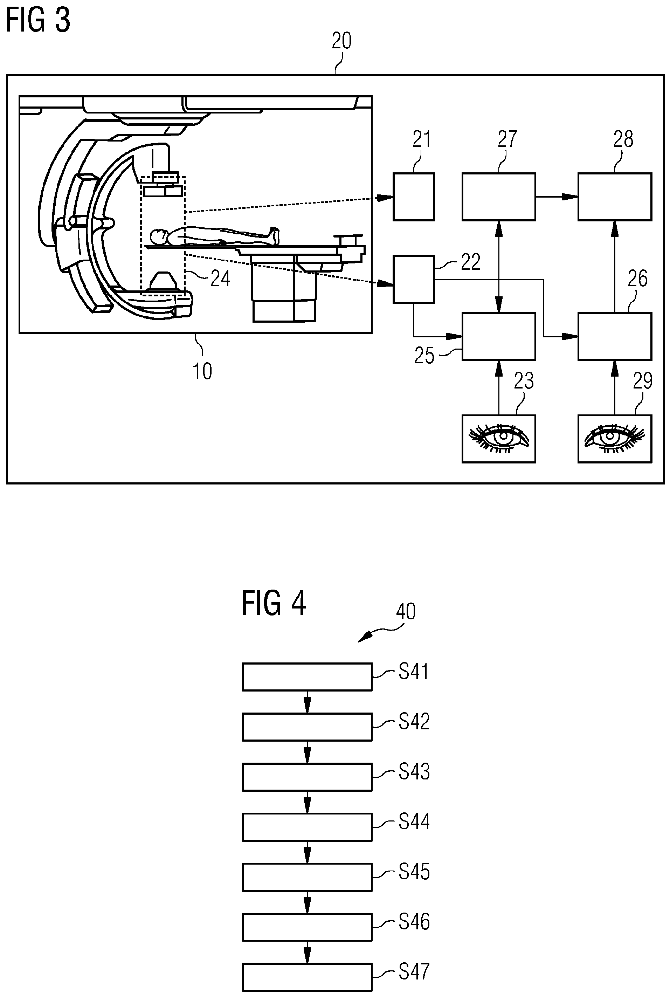

FIG. 3 illustrates a schematic diagram of an example of generating the stereoscopic medical image.

In this example of generating the stereoscopic medical image 20, the autostereoscopic display 11 is based on two-view systems, which provides a separate view for each eye of human. The autostereoscopic display 11 is configured to display the stereoscopic medical image, which the stereoscopic medical image integrated by the first autostereoscopic medical image 27 under the first autostereoscopic orientation 23 for the autostereoscopic display 11 and the second autostereoscopic medical image 28 under the second autostereoscopic orientation 29 for the autostereoscopic display 11. The image apparatus is a C-arm machine.

The 2D medical image 21 of a region of interest under an acquisition angulation 24 is captured. The 3D medical image 22 of the region of interest is captured. The acquisition angulation 24 of the 2D medical image 21 is to be used as a first autostereoscopic orientation 23.

The 2D medical image 21 and the 3D medical image 22 are co-registered under the acquisition angulation 24 to obtain a co-registration result. During the co-registration process, the depth of each 2D medical image 21 pixel is matched to the depth map of the 3D medical image 22 projected under the acquisition angulation 24.

The first volume projection 25 of the 3D medical image 22 is obtained under the acquisition angulation 24 based on the co-registration result to obtain a first autostereoscopic image under the first autostereoscopic orientation 23. Different volume rendering techniques can be applied to refine the correspondence between the 2D medical image 21 and the 3D medical image 22.

The second autostereoscopic orientation 29 is computed according to a location and/or an orientation of eyes of an external interacted user relative to the autostereoscopic display 11 and/or the first autostereoscopic orientation 23.

The second volume projection 26 of the 3D medical image 22 is computed under the second autostereoscopic orientation 29.

The 2D medical image 21 and the 3D medical image 22 under the second autostereoscopic orientation 29 are co-registered based on the second volume projection 26 by performing a pixel shifting on the 2D medical image 21 to obtain a second autostereoscopic medical image 28 under the second autostereoscopic orientation 29.

FIG. 4 illustrates a chart of an example of a method for enhancing depth perception.

The method 40 can be employed in the medical imaging apparatus 10 provided. The method 40 comprises acquiring S41 a 2D medical image 21 and a 3D medical image 22 of a region of interest under an acquisition angulation 24, which the acquisition angulation 24 is configured to use as a first autostereoscopic orientation 23; co-registering S42 the 2D medical image 21 and the 3D medical image 22 under the acquisition angulation 24 to obtain a co-registration result; computing S43 a second autostereoscopic orientation 29 for an autostereoscopic display 11 according to a location and/or an orientation of eyes of an external interacted user relative to the autostereoscopic display 11 and/or the first autostereoscopic orientation 23; obtaining S44 the first autostereoscopic image under the first autostereoscopic orientation 23 by computing a first volume projection 25 of the 3D medical image 22 under the acquisition angulation 24 based on the co-registration result; computing S45 a second volume projection 26 of the 3D medical image 22 under the second autostereoscopic orientation 29; obtaining S46 the second autostereoscopic image under the second autostereoscopic orientation 29 by co-registering the 2D medical image 21 and the 3D medical image 22 under the second autostereoscopic orientation 29 based on the second volume projection 26 by performing a pixel shifting on the 2D medical image 21; and displaying S47 a stereoscopic medical image integrated by a first autostereoscopic medical image 27 under the first autostereoscopic orientation 23 for the autostereoscopic display 11 and a second autostereoscopic medical image 28 under the second autostereoscopic orientation 29 for the autostereoscopic display 11.

Although the present invention has been described in the above by way of embodiments, it is not limited thereto, but rather can be modified in a wide range of ways. In particular, the invention can be changed or modified in various ways without deviating from the core of the invention.

Independent of the grammatical term usage, individuals with male, female or other gender identities are included within the term.

## Claims

1. A medical imaging apparatus (10) for enhancing depth perception during medical image guidance, the medical imaging apparatus (10) comprising:
an autostereoscopic display (11) configured to display a stereoscopic medical image, wherein the stereoscopic medical image is integrated by at least two autostereoscopic medical images;
an image-acquisition module (12) coupled to the autostereoscopic display (11) and configured to capture and/or receive a two-dimensional, 2D, medical image (21) of a region of interest under an acquisition angulation (24), and to capture and/or receive a three-dimensional, 3D, medical image (33) of the region of interest, wherein the acquisition angulation (24) of the 2D medical image (21) is to be used as a first autostereoscopic orientation (23);
a first registration module (13) coupled to the image-acquisition module (12) and configured to co-register the 2D medical image (21) and the 3D medical image (22) under the acquisition angulation (24) to obtain a co-registration result;
an image-derivation module (14) coupled to the autostereoscopic display (11) and the image-acquisition module (12) and configured to compute a second autostereoscopic orientation (29) according to a location and/or an orientation of eyes of an external interacted user relative to the autostereoscopic display (11) and/or the first autostereoscopic orientation (23);
a projection unit (15) coupled to the first registration module (13) and the image-derivation module (14) and configured to compute a first volume projection (25) of the 3D medical image (22) under the acquisition angulation (24) based on the co-registration result to obtain a first autostereoscopic medical image (27) under the first autostereoscopic orientation (23), and configured to compute a second volume projection (26) of the 3D medical image (22) under the second autostereoscopic orientation (29); and
a second registration module (16) coupled to the projection unit (15) and configured to co-register the 2D medical image (21) and the 3D medical image (22) under the second autostereoscopic orientation (29) based on the second volume projection (26) by performing a pixel shifting on the 2D medical image (21) to obtain a second autostereoscopic medical image (28) under the second autostereoscopic orientation (29).

2. The medical imaging apparatus according to claim 1, wherein the autostereoscopic display (11) is further configured to display the stereoscopic medical image, whereby the stereoscopic medical image is integrated by the first autostereoscopic medical image (27) under the first autostereoscopic orientation (23) for the autostereoscopic display (11) and the second autostereoscopic medical image (28) under the second autostereoscopic orientation (29) for the autostereoscopic display (11).

3. The medical imaging apparatus of according to any of the preceding claims, wherein the image-acquisition module (12) is configured such that the 2D medical image (21) comprises a 2D roadmap image.

4. The medical imaging apparatus according to any of the preceding claims, wherein the image-acquisition module (12) is configured such that the 3D medical image (22) comprises a 3D digitally subtracted angiographs, DSA.

5. The medical imaging apparatus according to any of the preceding claims, wherein the first registration module (13) is configured to co-register the 2D medical image (21) and the 3D medical image (22) under the acquisition angulation (24) via a geometry calibration or an image registration.

6. The medical imaging apparatus according to any of the preceding claims, wherein the first registration module (13) and the projection unit (15) are configured such that the first volume projection (25) of the 3D medical image (22) under the acquisition angulation (24) is represented by a correspondence between the depth of each 2D medical image (21) pixel and depth map of the 3D medical image (22) projected under the acquisition angulation (24), wherein the correspondence is refined by applying at least one of the volume rendering techniques, VRT, as follow:
- feature-based VRT;
- intensity-based VRT;
- gradient-based VRT; and/or
- combinations thereof.

7. The medical imaging apparatus according to any of the preceding claims, wherein the second registration module (16) is further configured to co-register the 2D medical image (21) and the 3D medical image (22) under the second autostereoscopic orientation (29) based on the second volume projection (26) by performing a depth-aware pixel shifting on the 2D medical image (21).

8. The medical imaging apparatus according to any of the preceding claims, wherein the medical imaging apparatus comprises further an eye tracking system (17) mounted on the autostereoscopic display (11), wherein the eye tracking system (17) is configured to obtain the location and the orientation of the eyes of the interacted user relative to the autostereoscopic display (11).

9. The medical imaging apparatus according to any of the preceding claims, wherein the eye tracking system (17) is further configured such that, when the location and/or the orientation of the eyes of the interacted user relative to the autostereoscopic display (11) is updated, an updated first autostereoscopic orientation (23) is computed based on a shift between a former location and/or the orientation of the eyes of the interacted user and an updated location and/or the orientation of the eyes of the interacted user.

10. The medical imaging apparatus according to any of the preceding claims, wherein the autostereoscopic display (11) is further configured to display a stereoscopic medical image integrated by multiple autostereoscopic medical images under corresponding autostereoscopic orientations.

11. The medical imaging apparatus according to any of the preceding claims, wherein the autostereoscopic display (11) is further configured to enable multi-observers to observe the region of interest displayed with the stereoscopic medical image.

12. The medical imaging apparatus according to any of the preceding claims, wherein the apparatus comprises as least one of the following apparatuses:
- mobile C-arms;
- X-ray machine; and/or
- computed tomography angiography, CTA, machine.

13. A computer-implemented method for enhancing depth perception during medical image guidance, in particular employed in a medical imaging apparatus (10) according to any of the claims 1 to 12, the method comprising:
acquiring (S41) a 2D medical image (21) and a 3D medical image (22) of a region of interest under an acquisition angulation (24), wherein the acquisition angulation (24) is configured to use as a first autostereoscopic orientation (23);
co-registering (S42) the 2D medical image (21) and the 3D medical image (22) under the acquisition angulation (24) to obtain a co-registration result;
computing (S43) a second autostereoscopic orientation (29) for an autostereoscopic display (11) according to a location and/or an orientation of eyes of an external interacted user relative to the autostereoscopic display (11) and/or the first autostereoscopic orientation (23);
obtaining (S44) the first autostereoscopic image under the first autostereoscopic orientation (23) by computing a first volume projection (25) of the 3D medical image (22) under the acquisition angulation (24) based on the co-registration result;
computing (S45) a second volume projection (26) of the 3D medical image (22) under the second autostereoscopic orientation (29);
obtaining (S46) the second autostereoscopic image under the second autostereoscopic orientation (29) by co-registering the 2D medical image (21) and the 3D medical image (22) under the second autostereoscopic orientation (29) based on the second volume projection (26) by performing a pixel shifting on the 2D medical image (21); and
displaying (S47) a stereoscopic medical image integrated at least by a first autostereoscopic medical image under the first autostereoscopic orientation (23) for the autostereoscopic display (11) and a second autostereoscopic medical image (28) under the second autostereoscopic orientation (29) for the autostereoscopic display (11).

14. A non-transitory computer-readable storage medium storing instructions by a computer that the instructions are implemented in the medical imaging apparatus of any of the claims 1 to 12.

15. A computer program product including a computer program that is implemented in the medical imaging apparatus of any of the claims 1 to 12 when program sections of the computer program are executed by a computer.
